(19) Europäisches Patentamt
European Patent Office
Office européen des brevets

(11) Veröffentlichungsnummer : **0 085 320**
**B1**

(12) # EUROPÄISCHE PATENTSCHRIFT

(45) Veröffentlichungstag der Patentschrift : **12.11.86**

(21) Anmeldenummer : **83100281.1**

(22) Anmeldetag : **14.01.83**

(51) Int. Cl.⁴ : **C 07 D498/08**, C 07 D498/18, G 01 N 33/00 // (C07D498/08, 273:00, 273:00),(C07D498/18, 273:00, 273:00, 221:00)

(54) **Kaliumreagens und Verfahren zur Bestimmung von Kaliumionen.**

(30) Priorität : **28.01.82 DE 3202779**

(43) Veröffentlichungstag der Anmeldung : **10.08.83 Patentblatt 83/32**

(45) Bekanntmachung des Hinweises auf die Patenterteilung : **12.11.86 Patentblatt 86/46**

(84) Benannte Vertragsstaaten : **AT CH DE FR GB IT LI NL SE**

(56) Entgegenhaltungen : **EP-A- 0 010 615**

(73) Patentinhaber : **Merck Patent Gesellschaft mit beschränkter Haftung**
**Frankfurter Strasse 250**
**D-6100 Darmstadt (DE)**

(72) Erfinder : **Klink, Rainer, Dr.**
**Heidelberger Landstrasse 16**
**D-6100 Darmstadt-Eberstadt (DE)**
Erfinder : **Bodart, Detlef, Dr.**
**Caixa Postal 55.012**
**Rio de Janeiro (BR)**
Erfinder : **Lehn, Jean-Marie, Prof.**
**1, Rue Blaise Pascal**
**F-67008 Strasbourg Cdex (FR)**
Erfinder : **Helfert, Bernd**
**Schillerstrasse 1**
**D-6105 Ober-Ramstadt (DE)**
Erfinder : **Bitsch, Roland**
**Karl-Marx-Strasse 40**
**D-6102 Pfungstadt (DE)**

EP 0 085 320 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Farbreagens zur einfachen, sicheren und schnellen Bestimmung von Kaliumionen sowie das Verfahren zu dessen Verwendung.

Die qualitative und quantitative Bestimmung von Kaliumionen ist beispielsweise in der chemischen und biochemischen Technik zur Prozesskontrolle, in der Agrikulturchemie zur Bodenuntersuchung und Dosierung von Düngemitteln und in der Medizin etwa zur diagnostischen und therapeutischen Kontrolle des Kalium-Natrium-Verhältnisses von erheblicher und zunehmender Bedeutung. Außer der Flammenphotometrie und der Atomabsorptions-Spektroskopie, die beide apparativ sehr aufwendig sind, gibt es bis heute kaum zuverlässige Methoden zur Kaliumbestimmung. Die seit kurzem angebotenen ionensensitiven Kalium-Elektroden auf Ionenaustauscherbasis liefern gewöhnlich ungenügend differenzierte Resultate.

Einfache und gleichzeitig zuverlässige Methoden zur qualitativen und quantitativen Bestimmung von Kalium fehlen. Es gibt beispielsweise bisher kein brauchbares Farbreagens für Kaliumionen.

In der deutschen Offenlegungsschrift (DE-OS) 2 842 862 ist ein Verfahren zur Bestimmung von Ionen beschrieben, welches im Prinzip auf der ionenselektiven Komplexbindung zwischen dem zu bestimmenden Ion und einem Komplexbildner und der Messung der bei der Komplexbildung auftretenden Extinktionsänderung beruht, wobei der Komplexbildner mit einem Farbträger verbunden ist.

Der selektive Komplexbildner kann ein Oligo-ether, -ester oder -amid sein, beispielsweise ein Kronenether, Cryptand oder cyclisches Peptid und/oder Polyethylenglykolgruppierungen oder andere Heteroatome-haltige Gruppierungen enthalten. Der kovalent oder heteropolar gebundene Farbträger ist ein Farbstoff oder Fluoreszenzfarbstoff oder ein Chromogen, dessen Absorptionsspektrum sich durch Wecheslwirkung mit apolaren Ionen oder lipophilen Molekülen durch Ladungsverschiebung oder Störung des mesomeren Systems ändert.

Dieses Prinzip ist in der Natur und der Technik gut bekannt. Hämin, Chlorophyll und etwa Metallkomplexfarbstoffe sowie Metallindikatoren (z. B. Xylenolorange und Methylthymolblau auf der Basis des farblosen Komplexbildners EDTA) entsprechen mehr oder weniger diesem generellen Bauplan.

Die in der DE-OS 2 842 862 offenbarten Beispiele erfüllen die Anforderungen der Praxis hinsichtlich der Bestimmung von Kalium nicht. Es konnten danach mit Kaliumionen keine brauchbaren Farbreaktionen erhalten werden.

Ein generelles Problem der vorstehenden Komplexbildner ist, daß sie gewöhnlich nur in organischen Medien zur Reaktion befähigt sind, das zu bestimmende Ion dagegen in aller Regel in wäßriger Lösung vorliegt. Zwar würden sich die wäßrigen Lösungen der Ionen durch Eindampfen, Einbau in organische Salze oder Lösungsmittelextraktion in vielen Fällen in organische Medien überführen lassen, dies würde jedoch nicht den Anforderungen einer praktischen und bei Bedarf automatisierbaren Schnellmethode entsprechen.

Der Erfindung lag daher die Aufgabe zugrunde, ein zuverlässiges Reagens zur Bestimmung von Kaliumionen zu entwickeln, das sich einfach verwenden läßt, Schnellbestimmungen und auch Bestimmung von Kaliumionen in einphasigen wäßrigen Medien gestattet.

Es wurde nun gefunden, daß bestimmte neue Cryptanden eine hohe Selektivität für die Komplexbildung von Kalium aufweisen und, soweit sie mit Chromophoren verknüpft sind, intensive, analytisch auswertbare Farbreaktionen geben.

Gegenstand der Erfindung sind demnach Verbindungen der allgmeinen Formel I

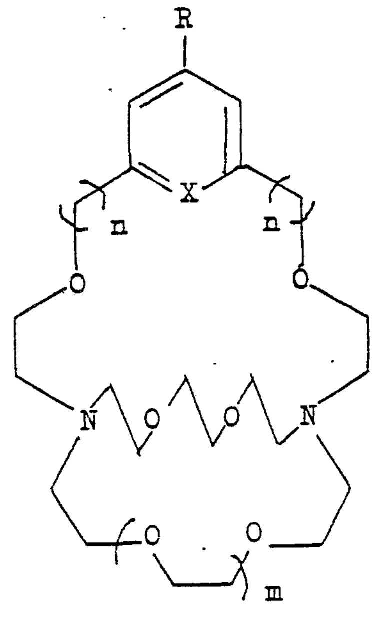

(I)

Auch aliphatische Alkohole wie Methanol und Ethanol sind prinzipiell geeignet, bessere Resultate werden jedoch in Alkoholen mit 3-8 C-Atomen wie Isopropanol, n-Propanol, Butanolen, Amylalkoholen, Hexanolen, Heptanolen und Octanolen erzielt. Auch Dimethylsulfoxid ist ein geeignetes Lösungsmittel. Als Aminoalkohole eignen sich insbesondere Ethanolamin, Propanolamin, Amino-propandiole sowie N-Methylglucamin. Als besonders vorteilhaft erwies sich das Lösungsmittelsystem Wasser/Dioxan/Morpholin.

Die Farbintensität der Kalium-Komplexe der allgemeinen Formel I nimmt mit steigendem Wasseranteil des Mediums ab. Zur Erzielung einer photometrisch und visuell auswertbaren Farbdifferenz zwischen Blind- und Probenwert ist ein Mischungsverhältnis Wasser/organisches Lösungsmittel von etwa 1 : 4 bis 1 : 6 erstrebenswert, d. h. auf 1 Teil wäßriger Probelösung werden etwa 4-6 Teile Dioxan bzw. Methanol usw. eingesetzt. Wird der Wasseranteil erheblich reduziert, so treten Meßwertstreuungen auf. Von Fall zu Fall wird das Mischungsverhältnis dahingehend optimiert, daß einerseits der Anteil der wäßrigen Phase zwecks gesteigerter Empfindlichkeit möglichst hoch gehalten wird, auf der anderen Seite der organische Anteil noch eine Farbreaktion mit gutem Reaktionskontrast sichert. Der Ansatz in Dioxan zeigt z. B. eine deutliche bessere Farbabstufung als eine Probe gleicher Konzentration in Methanol.

Die in der DE-OS 2 842 862 offenbarten Kaliumreagentien zeigten den oben beschriebenen überraschenden, vom Medium abhängigen Farbeffekt, der diese Reaktion überhaupt erst brauchbar macht, nicht.

Der aufgrund von zahlreichen Erfahrungen mit Kaliumreagentien vermutete störende Einfluß von neben Kalium in der Praxis oft gleichzeitig vorkommenden verwandten Ionen, darunter auch solchen mit im allgemeinen weit höherer Komplexbildungstendenz, trat überraschenderweise nicht auf, wie die nachstehenden Extinktionsmaxima demonstrieren.

Extinktionsmaxima der Alkali- und Erdalkalimetall-Komplexe einer Verbindung der Allgemeinen Formel I worin n = 0, m = 1, X = COH, R = 4-Nitrophenylazo bedeutet.

| Kation | Extinktionsmaxima in nm |
|---|---|
| Kalium | 570 |
| Natrium | 510 |
| Lithium | 515 |
| Rubidium | 567 |
| Cäsium | 500 |
| Calcium | 510 |
| Strontium | 517 |
| Magnesium | 425 |
| Barium | 530 |

Während das praktisch wenig bedeutende Rubidium ($E_{max}$ 567 nm) stört, liegen alle andern Extinktionsmaxima so weit entfernt, daß eine quantitative Bestimmung von Kalium mit jedem Spektralphotometer möglich ist.

Die Extinktionsmessung ergab bei gleichen Konzentrationen von Rubidium und Kalium ein Verhältnis von 1 : 2,5.

Verbindungen der allgemeinen Formel I, worin n = 0, m = 1, X = COH und R =

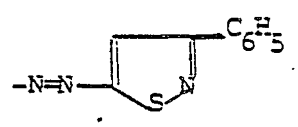

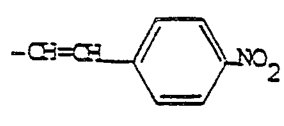

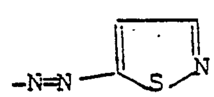

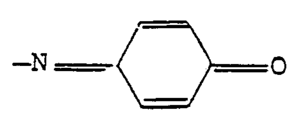

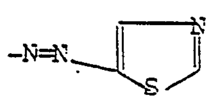

worin

n = 0 oder 1, m = 0 oder 1

X = N oder COH

R =

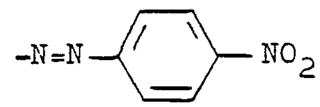

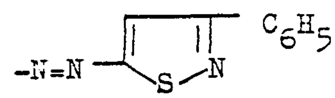

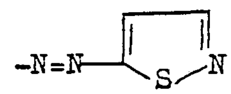

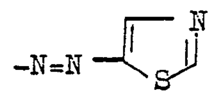

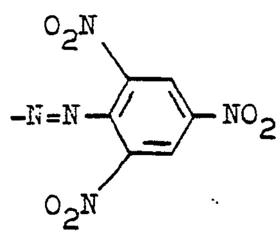

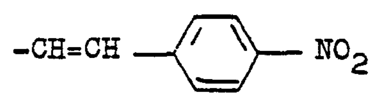

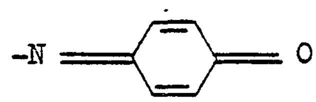

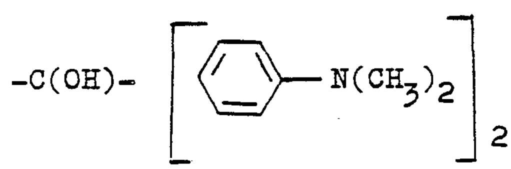

bedeuten,

Ihre Farbintensität und Stabilität läßt eine einfache reproduzierbare, quantitative Kaliumbestimmung zu.

Ein weiterer Gegenstand der Erfindung ist ein Verfahren zur Bestimmung von Kalium mit Hilfe von Verbindungen der allgemeinen Formel I, das allgemein dadurch gekennzeichnet ist, daß die Umsetzung der zu bestimmenden Kaliumionen in Flüssigkeiten in einem Reaktionsmedium aus Wasser und mindestens einem mit Wasser mischbaren organischen Lösungsmittel und in Gegenwart einer organischen Base erfolgt und anschließend die Extinktionsänderung bestimmt wird.

Es zeigte sich, daß eine große Anzahl von mit Wasser mischbaren organischen Lösungsmitteln wie beispielsweise Aceton, Methylethylketon und Eisessig als Reaktionsmedien ungeeignet sind. Überraschend wurde gefunden, daß in cyclischen Ethern, Glykolethern, Amiden, aliphatischen Alkoholen mit vorzugsweise 3 bis 8 C-Atomen und/oder Sulfoxiden in Gegenwart von Morpholin und oder Aminoalkoholen ausgezeichnete sowohl photometrisch als auch visuell verwertbare Farbabstufungen erhalten werden. .

Als cyclische Ether sind insbesondere Dioxan und Tetrahydrofuran, als Glykolether Ethylenglykolmono-alkylether, insbesondere Methyl-, Ethyl-, Propyl- und Butylcellosolve und als Amide Formamid, Dimethylformamid (DMF), Pyrrolidon und N-Alkyl-pyrrolidone, z. B. N-Methylpyrrolidon (NMP), geeignet.

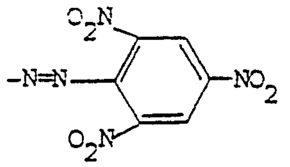

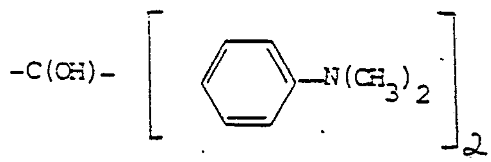

bedeuten,

verhalten sich analog wie die obengenannte Verbindung der allgemeinen Formel I, worin n = 0, m = 1, X = COH und R = 4-Nitrophenylazo bedeuten. Auch die analogen Verbindungen der allgemeinen Formel I, in denen X = N bedeutet, geben mit Kaliumionen ebenfalls analytisch auswertbare Farbreaktionen. Die Verbindungen der allgemeinen Formel I, in denen sowohl n als auch m 1 bedeutet, verhalten sich auch analog wie die vorgenannten Verbindungen und geben mit Kaliumionen auswertbare Farbreaktionen.

Im Falle X = N tritt eine hypsochrome Verschiebung des Absorptionsmaximums bei der Wechselwirkung mit dem Kation ein, da die Verfügbarkeit des freien Elektronenpaars am Stickstoff durch die Komplexierung ganz oder teilweise gebunden wird und es nicht mehr zur Mesomerie mit dem Chromophor zur Verfügung steht. Im Falle X = COH tritt der umgekehrte Fall, nämlich eine bathochrome Verschiebung nach erfolgter Komplexierung auf.

Neben dem Verfahren zur spektrophotometrischen Bestimmung von Kaliumionen ist es auch möglich, zur Trennung und Bestimmung von verschiedenen anderen Kationen eine mit einer Verbindung der allgemeinen Formel I imprägnierte Dünnschichtchromatographieplatte zu verwenden bzw. eine Dünnschichtchromatographieplatte nach der Entwicklung mit einem entsprechenden Reagenz zu besprühen.

Die Herstellung der neuen Verbindungen der allgemeinen Formel I erfolgt nach an sich bekannten Methoden, wie sie in der Literatur [z. B. in den Standardwerken wie Houben-Weyl, Methoden der organischen Chemie, Georg Thieme Verlag Stuttgart ; Tetrahedron *29,* 1629-1645 (1973)] beschrieben sind, und zwar unter Reaktionsbedingungen, die für die genannten Umsetzungen bekannt und geeignet sind.

## Beispiel 1

Herstellung der Verbindung der allgemeinen Formel I, worin n = 0, m = 1, X = COH und R = N = N—Ph—$NO_2$ bedeute : 30-Hydroxy-7-(p-nitrophenylazo)-4.10.16.19.24.27-hexaoxa-1.13-diazatricyclo [11.8.8.$1^{5.9}$] triaconta-5.7.9-trien.

a) 1,3-Dioxydiessigsäure-2-hydroxy-benzol (1)

In 1 000 ml Wasser löst man 403 g (4,26 Mol) Chloressigsäure und fügt bei 0-5 °C die Lösung von 170 g (4,26 Mol) Natriumhydroxid in 350 ml Wasser zu. Man fügt 271 g (2,15 Mol) Pyrogallol zu und erwärmt die Mischung 4 Stunden unter Rückfluß. Während dieser Zeit gibt man die Lösung von 180 g Natriumhydroxid in 400 ml Wasser portionsweise so zu, daß das Reaktionsgemisch immer alkalisch reagiert. Am nächsten Tag fügt man Salzsäure bis zur stark sauren Reaktion zu, kühlt auf 0-5° ab und trennt den ausgefallenen Niederschlag ab. Nach dem Umkristallisieren aus Wasser erhält man 53 g (1) von Schmp. 176-178 °C.

b) 1,3-Dioxydiessigsäure-2-methoxy-benzol (2)

48 g (0,2 Mol) (1) löst man in einer Lösung von 28 g Natriumhydroxid in 200 ml Wasser. Bei 10-20 °C tropft man unter Kühlung 65 ml (0,68 Mol) Dimethylsulfat innerhalb 1 Stunde zu und rührt das Gemisch 20 Stunden bei Raumtemperatur, wobei sich aus der zunächst klaren Lösung allmählich ein Öl abscheidet. Man erwärmt 1 Stunde unter Rückfluß, kühlt auf 5-10 °C ab und fügt konz. Salzsäure bis zur stark sauren Reaktion zu. Man extrahiert viermal mit je 250 ml Ethylacetat und gewinnt nach Entfernen des Lösungsmittels ein Produkt, das in einer Lösung von 20 g Natriumhydroxid in 200 ml Wasser 5 Stunden unter Rückfluß erwärmt wird. Man kühlt dann auf 35-40 °C ab und fügt konz. Salzsäure bis zur stark sauren Reaktion zu. Nach dem Kühlen auf 0 °C saugt man den auskristallisierten Niederschlag ab und erhält nach dem Trocknen 35 g vom Schmp. 150-152 °C.

c) 1,3-Dioxydiessigsäurechlorid-2-methoxy-benzol (3)

83 g (0,33 Mol) (2) werden in 500 ml Chloroform aufgeschlämmt. In die kochende Suspension tropft man innerhalb von 1 Stunde 100 ml (1,38 Mol) Thionylchlorid zu und kocht die Mischung über Nacht unter Rückfluß. Überschüssiges Thionylchlorid und Lösungsmittel werden im Vakuum entfernt. Als Rückstand verbleiben 94 g kristallines (3).

d) 30-Methoxy-4.10.16.19.24.27-hexaoxa-1,13-diaza-tricyclo [11.8.8.$1^{5.9}$] triaconta-5.7.9-trien-2.12-dion (4)

Zu 1,5 l Toluol gibt man gleichzeitig und mit gleicher Zulaufgeschwindigkeit die Lösung von 77 g (0,26 Mol) (3) in 2,0 l Toluol und die Lösung von 68 g (0,26 Mol) 4.7.13.16-Tetraoxa-1.10-diaza-cyclooctadecan und 59 g Triethylamin in 2 l Toluol über einen Zeitraum von 3-4 Stunden unter kräftigem Rühren. Man erwärmt auf 70 °C und saugt vom Unlöslichen ab. Das Filtrat wird im Vakuum bis zur Trockne eingeengt. Der Rückstand (57 g) wird mit 1 l Ethylacetat erschöpfend extrahiert. Der Extrakt wird im Vakuum eingeengt und das ausgefallene Produkt isoliert (52 g).

e) 30-Methoxy-4.10.16.19.24.27-hexaoxa-1.13-diaza-tricyclo [11.8.8.$1^{5.9}$] triaconta-5.7.9-trien (5)

52 g (0,107 Mol) (4) trägt man in eine Lösung von 0,25 Mol Diboran in 500 ml Tetrahydrofuran ein. Die Mischung wird 6 Stunden bei Raumtemperatur gerührt und dann 15 Stunden unter Rückfluß erhitzt. Man destilliert im Vakuum das Lösungsmittel vollständig ab und extrahiert den Rückstand mit Chloroform. Dieser Extrakt wird bis zur Trockne eingeengt und der Rückstand 6 Stunden mit 6 N Salzsäure unter Rückfluß erhitzt. Man dampft zur Trockne ein, nimmt den Rückstand in wenig Wasser auf und filtriert diese Lösung über einen stark basischen Ionenaustauscher. Das Eluat wird zur Trockne eingedampft und der feste Rückstand aus Petroleumbenzin (50-70 °C) umkristallisiert. Man gewinnt 44 g (5) vom Schmp. 86-88 °C.

f) 30-Hydroxy-4.10.16.19.24.27-hexaoxa-1.13-diaza-tricyclo [11.8.8.$1^{5.9}$] triaconta-5.7.9-trien (6)

12 g trockenes Lithiumjodid löst man in 50 ml Pyridin und gibt 12 g (5) zu. Man hält die Lösung 15 Stunden bei 90-100 °C. Nach Entfernung des Pyridins im Vakuum nimmt man den Rückstand in Wasser auf und fügt unter Kühlung und Rühren 32 %ige Natronlauge bis zur stark alkalischen Reaktion zu, wobei sich ein Öl abscheidet, das bald erstarrt. Das Festprodukt wird isoliert und zweimal aus Wasser umkristallisiert (11,8 g). Dieses Produkt ist das Na-Salz von (6).

g) 30-Hydroxy-7-(p-nitrophenylazo)-4.10.16.19.24.27-hexaoxa-1.13-diaza-tricyclo [11.8.8.$1^{5.9}$] triaconta-5.7.9-trien (7)

5 g (6) löst man in 100 ml Eisessig und fügt innerhalb 1 Stunde die Lösung von 1,4 g diazotiertem 4-Amino-1-nitrobenzol hinzu. Es bildet sich eine rotbraune Lösung, die über Nacht bei Raumtemperatur aufbewahrt wird. Man engt im Vakuum bis zur Trockne ein, nimmt den Rückstand in 250 ml Wasser auf und fügt Natronlauge bis zur alkalischen Reaktion zu. Es scheidet sich ein sehr feiner violetter Niederschlag ab, der in Toluol aufgenommen wird (fünfmaliges Extrahieren mit je 250 ml Toluol). Die Extrakte werden mit Wasser gewaschen, wobei sie eine orangegelbe Farbe annehmen. Nach Entfernen des Toluols im Vakuum wird der feste Rückstand aus Methanol/Wasser umkristallisiert. Man erhält 2 g rotbraunes Kristallisat (Na-Salz von (7)) von undeutlichem Schmelzverhalten : Sintern bei 94-96 °C und Schmelzen bzw. Zersetzung bei 98-102 °C.

C : ber. 56,2 %, gef. 56,9 % ; H : ber. 6,4 % gef. 6,5 % ;

N : ber. 11,8 %, gef. 11,8 %.

Analog Beispiel 1 g) werden die entsprechenden Azoverbindungen hergestellt, die in der 7-Stellung anstelle von p-Nitrophenylazo die Reste 3-Phenylisothiazolyl-5-azo, Isothiazolyl-5-azo, Thiazolyl-5-azo, 2.4.6-Trinitrophenylazo und p-Nitrostyryl haben.

Die Verbindungen der allgemeinen Formel I, worin R = p-Nitrostyryl bedeutet, werden erhalten durch Umsetzung von Verbindungen der allgemeinen Formel I, worin R = $-CH_3$ bedeutet, mit p-Nitrobenzaldehyd.

Verbindungen der allgemeinen Formel I, worin R = p-Benzochinonmonoimino bedeutet, werden erhalten aus entsprechenden 4-Amino-Verbindungen durch Kondensation mit Benzochinon. Die Bis-(p-dimethylaminophenyl)-hydroxymethyl-Derivate schließlich werden erhalten durch Kondensation von Michlers Keton mit Verbindungen der allgemeinen Formel I, worin R Wasserstoff bedeutet.

## Beispiel 2

1 ml Probelösung enthaltend 10 ppm Kaliumionen wird mit 5 ml Dioxan und 0,2 ml Morpholin gemischt. 0,2 ml einer 0,05 %igen Stammlösung der Verbindung der allgemeinen Formel I, worin n = 0, m = 1, X = COH und R = 4-Nitro-phenylazo bedeutet, in Dimethylsulfoxid werden zugefügt. Parallel dazu wird mit einer Blindprobe mit destilliertem Wasser anstelle der Kalium-haltigen Probelösung in gleicher Weise verfahren. Bei der Probelösung tritt ein Farbumschlag von rosa in gentianaviolett ein. Die Absorptionsspektren (1) von Probe (Pr) gegen Blindprobe (Bl), (2) Probe gegen Wasser (Pr : Wa) und (3) von Blindprobe gegen Wasser (Bl : Wa) werden bestimmt. Siehe Tabelle 2.

### Beispiel 3

1 ml Probelösung mit 10 ppm Kaliumionen werden mit 5 ml Tetrahydrofuran und 0,2 ml Morpholin gemischt, 0,2 ml Stammlösung werden zugefügt. Ein Farbumschlag von rosa nach violett wird registriert. Eine Blindprobe wird analog bereitet. Die Absorptionsspektren von (1) Pr gegen Bl, (2) Pr gegen Wa und (3) Bl gegen Wa werden bestimmt. Siehe Tabelle 2.

### Beispiel 4

1 ml Probelösung mit 10 ppm Kaliumionen werden mit 5 ml Isopropanol und 0,2 ml einer Mischung aus 3 Teilen Morpholin und 1 Teil Ethanolamin versetzt und mit 0,2 ml der im Beispiel 1 beschriebenen Stammlösung vermischt. Es erfolgt ein Farbumschlag von graurosa nach stahlblau. Eine Blindprobe mit destilliertem Wasser wird gleich behandelt.

Die Absorptionsspektren werden bestimmt. Siehe Tabelle 2.

### Beispiel 5

Die Probelösung (1 ml mit 10 ppm Kaliumionen) wird mit 5 ml Morpholin gemischt, 0,2 ml Stammlösung werden zugefügt. Es erfolgt ein Farbumschlag von hellgraublau nach tiefblau.

Eine Blindprobe wird analog bereitet. Die Spektren werden aufgenommen. Siehe Tabelle 2.

### Beispiel 6

Die Probelösung (1 ml mit 10 ppm Kaliumionen) wird mit 5 ml Ethylenglykolmonoethylether und 0,2 ml einer Mischung aus Morpholin und Ethanolamin (3 : 1) vermischt. 0,2 ml Stammlösung werden zugesetzt.

Ein Farbumschlag von hellrotbraun nach blau wird beobachtet. Das Ergebnis zeigt die nachstehende Tabelle :

Tabelle 2 : Auswertung der Absorptionsspektren von Kalium-Probelösungen und Blindproben (Pr = Probelösung, Bl = Blindprobe, Wa = Wasser) der Beispiele 1-5.

| Beispiel | Extinktionsmaxima | | | Extinktion E bei $E_{max}$ | | | E von Bl bei |
|---|---|---|---|---|---|---|---|
| Nr. | Pr:Wa | Pr:Bl | Bl:Wa | Pr:Wa | Pr:Bl | Bl:Wa | $E_{max}$ v. Pr. |
| 1 | 562 | 570 | 412 | 0.34 | 0.305 | 0.035 | 0.065 |
| 2 | 570 | 567 | 412/560 | 0.32 | 0.225 | 0.095 | 0.135 |
| 3 | 559 | 558 | 412 | 0.35 | 0.238 | 0.112 | 0.125 |
| 4 | 568 | 565 | 569 | 0.50 | 0.182 | 0.323 | 0.334 |
| 5 | 550 | 575 | 490 | 0.24 | 0.118 | 0.12 | 0.15 |

Die Absorptionsspektren von Probe- und Blindlösungen gegen Wasser und untereinander lassen klar erkennen, daß die Systeme für eine qualitative und auch quantitative Bestimmung von Kaliumionen hervorragend geeignet sind. Die Bestimmungsmethode ist, wie die Beispiele zeigen, einfach und, wie die Daten der Tabelle 2 beweisen, zuverlässig. Bei optimaler Wahl des Reaktionsmediums ist die Extinktion der Blindprobe im Bereich des Extinktionsmaximums der Meßprobe (Pr) gering und die Differenz der Extinktion zwischen Probe gegen Wasser und Probe gegen Blindwert ebenfalls gering (Beispiel 1-3). Die große Variationsbreite im Lösungmittelsystem erlaubt die Herstellung verschiedener, dem Verwendungszweck präzis angepaßter, Reagensformen.

### Beispiel 7

Prüfung der Linearität zwischen Extinktion und Gehalt an Kaliumionen.

Wäßrige Probelösungen von je 1 ml enthaltend 2,4,6,8 und 10 ppm Kaliumionen werden mit 5 ml Dioxan, 3 Tropfen Morpholin und 2 Tropfen Stammlösung analog Beispiel 1 versetzt. Die Extinktion gegen den Blindwert wurde bei E = 570 nm gemessen.

Tabelle 3

| ppm $K^{(+)}$ | E = 570 nm | E = 570 : ppm |
|---|---|---|
| 2 | 0.121 | 0.0605 |
| 4 | 0.25 | 0.0625 |
| 6 | 0.4 | 0.0666 |
| 8 | 0.54 | 0.0675 |
| 10 | 0.66 | 0.0660 |

Es besteht eine sehr gute, präzise Linearität zwischen Extinktion und Kaliumionen-Gehalt, welche selbst die quantitative Bestimmung von geringen Kaliumkonzentrationen erlaubt.

Beispiel 8

Die Probelösung (1 ml) wird mit 5 ml DMF und 0,3 ml N-2-Hydroxymethyl-morpholin und danach mit 0,2 ml Stammlösung vermischt. Ein Farbumschlag von rosa nach blau wird beobachtet.

Beispiel 9

Teststreifen

Filterpapierstreifen werden in eine 1 %ige Lösung von Verbindung I (n = 0, m = 1, X = COH, R = 4-Nitrophenylazo) in Dimethylsulfoxid getaucht, antrocknen gelassen, anschließend ausgiebig mit einer 25 %igen wäßrigen Lösung von N-Methylglucamin besprüht, erneut getrocknet, schließlich mit Octanol besprüht und erneut getrocknet. Beim Auftragen eines Tropfens einer wäßrigen Lösung enthaltend 20 ppm Kaliumionen tritt auf der Tropfstelle ein Farbumschlag von hellbeige nach violett auf.

**Patentansprüche** (für die Vertragsstaaten : CH, DE, FR, GB, IT, LI, NL, SE)

1. Verbindungen der allgemeinen Formel I

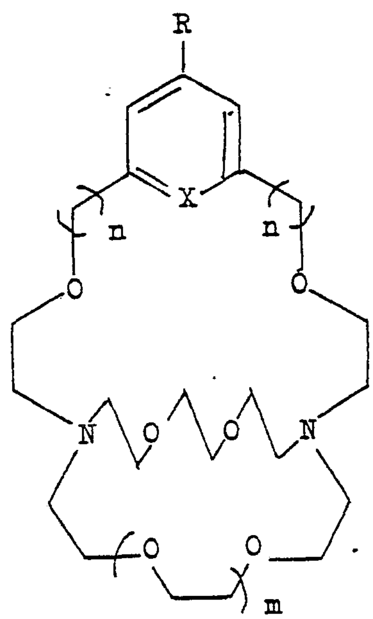

(I)

worin

n und m = 0 oder 1

X = N oder COH

R = 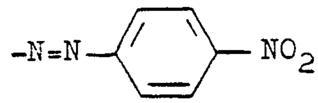

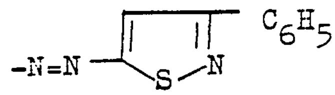

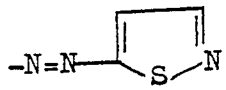

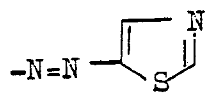

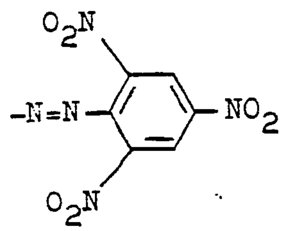

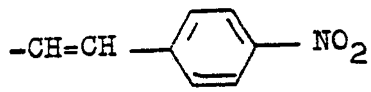

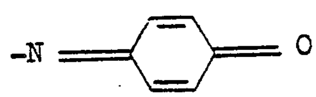

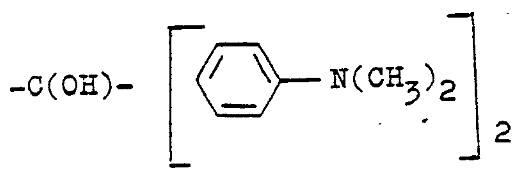

bedeuten.

2. Verfahren zur Bestimmung von Kaliumionen in Flüssigkeiten mit Hilfe von Verbindungen der allgemeinen Formel I, dadurch gekennzeichnet, daß die Umsetzung in einem Reaktionsmedium aus Wasser und mindestens einem mit Wasser mischbaren organischen Lösungsmittel der Reihe cyclische Ether, Glykolether, Amide, aliphatische Alkohole und/oder Sulfoxide und in Gegenwart von Morpholin und/oder eines Aminoalkohols erfolgt und anschließend die Extinktionsänderung bestimmt wird.

3. Verfahren nach Anspruch 2, dadurch gekennzeichnet, daß Wasser und organisches Lösungsmittel im Verhältnis 1 : 4 bis 1 : 6 verwendet werden.

**Patentansprüche** (für den Vertragsstaat AT)

1. Verfahren zur Bestimmung von Kaliumionen in Flüssigkeiten mit Hilfe von Verbindungen der allgemeinen Formel I,

(Siehe Schema Seite 10 f.)

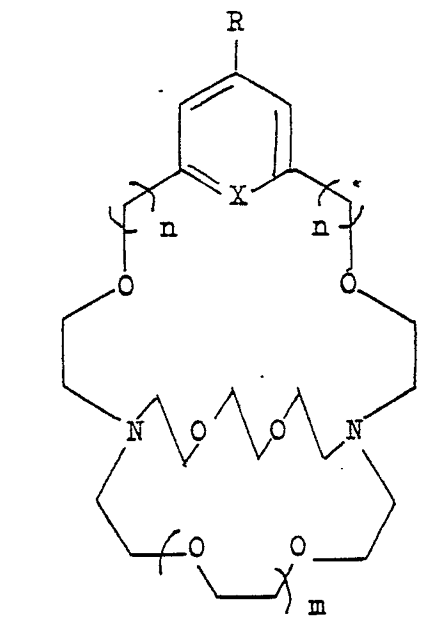

(I)

worin

n und m = 0 oder 1

X = N oder COH

R =

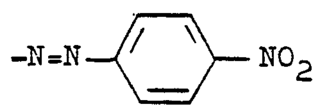

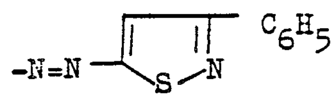

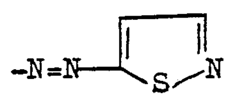

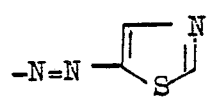

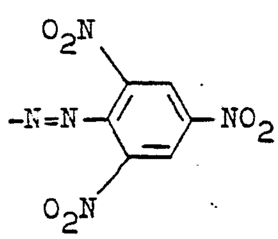

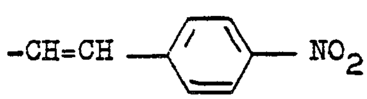

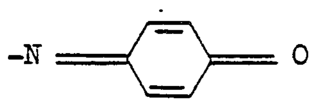

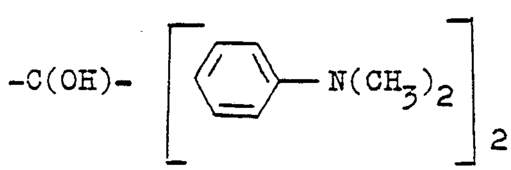

bedeuten,
dadurch gekennzeichnet, daß die Umsetzung in einem Reaktionsmedium aus Wasser und mindestens einem mit Wasser mischbaren organischen Lösungsmittel der Reihe cyclische Ether, Glykolether, Amide, aliphatische Alkohole und/oder Sulfoxide und in Gegenwart von Morpholin und/oder eines Aminoalkohols erfolgt und anschließend die Extinktionsänderung bestimmt wird.

2. Verfahren nach Anspruch 1, dadurch gekennzeichnet daß Wasser und organisches Lösungsmittel im Verhältnis 1 : 4 bis 1 : 6 verwendet werden.

**Claims** (for the Contracting States : CH, DE, FR, GB, IT, LI, NL, SE)

1. Compounds of the general formula I

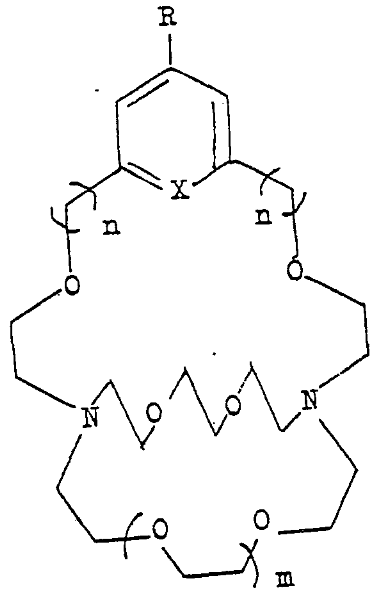

(I)

wherein
n and m are 0 or 1,
X is N or COH and
R is

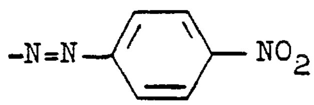

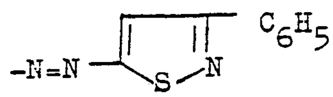

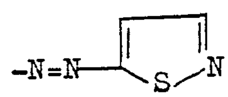

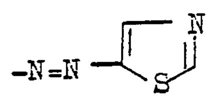

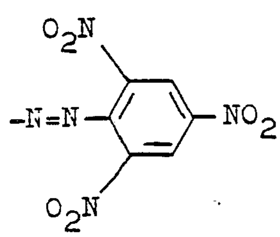

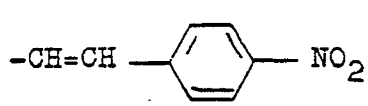

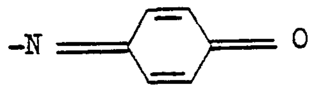

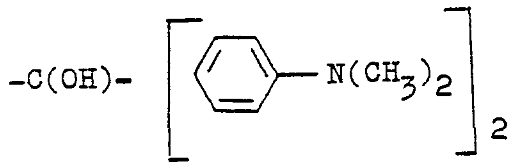

2. Process for the determination of potassium ions in liquids with the aid of compounds of the general formula I, characterised in that the reaction is carried out in a reaction medium of water and at least one water-miscible organic solvent of the group consisting of cyclic ethers, glycol ethers, amides, aliphatic alcohols and/or sulfoxides and in the presence of morpholine and/or an aminoalcohol and the change in extinction is then determined.

3. Process according to Claim 2, characterised in that the water and the organic solvent are used in a ratio of 1 : 4 to 1 : 6.

**Claims** (for the Contracting State AT)

1. Process for the determination of potassium ions in liquids with the aid of compounds of the general formula I

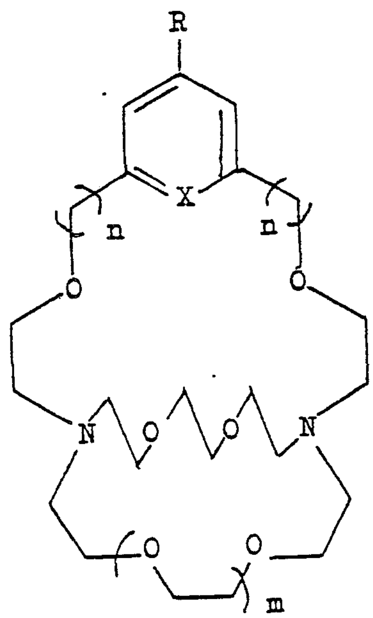

(I)

wherein

n and m are 0 or 1,
X is N or COH and
R is

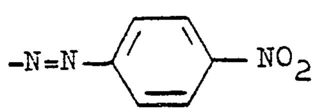

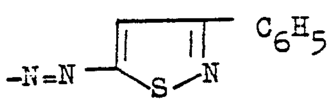

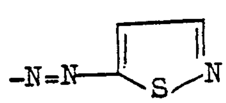

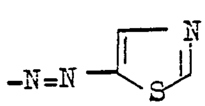

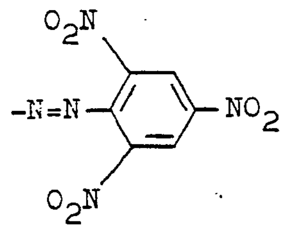

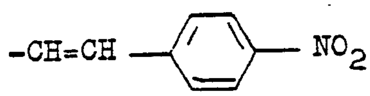

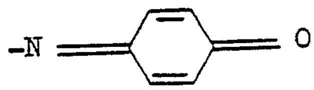

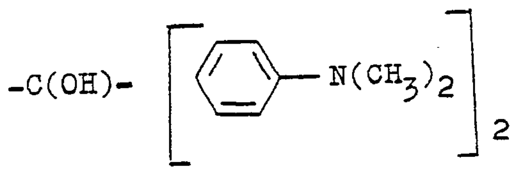

characterised in that the reaction is carried out in a reaction medium of water and at least one water-miscible organic solvent of the group consisting of cyclic ethers, glycol ethers, amides, aliphatic alcohols and/or sulfoxides and in the presence of morpholine and/or an aminoalcohol and the change in extinction is then determined.

2. Process according to claim 1, characterised in that the water and the organic solvent are used in a ratio of 1 : 4 to 1 : 6.

**Revendications** (pour les Etats contractants : CH, DE, FR, GB, IT, LI, NL, SE)

1. Composés de formule générale I

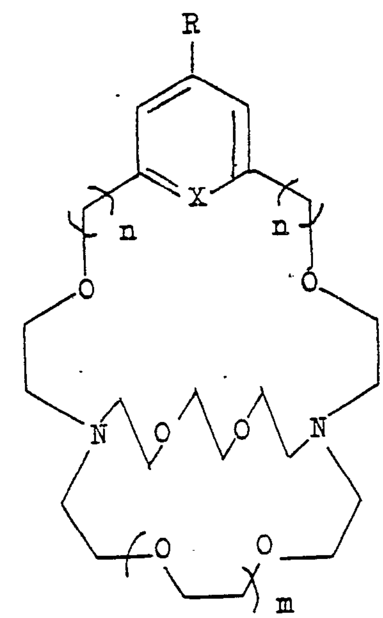

(I)

dans laquelle

n et m = 0 ou 1,

X = N ou COH

R =

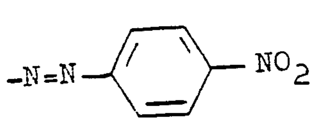

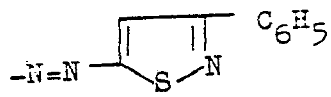

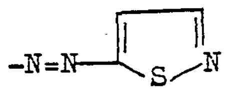

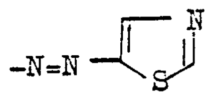

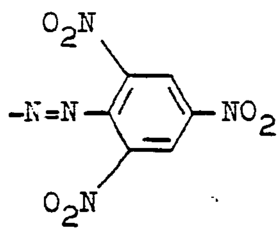

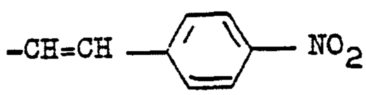

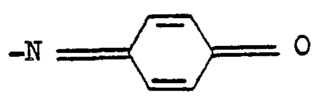

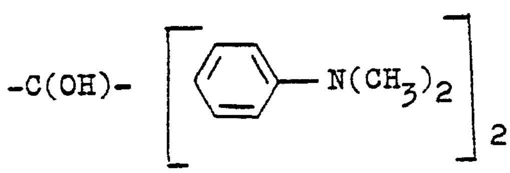

2. Procédé pour l'analyse des ions potassium dans des liquides à l'aide de composés de formule générale I, caractérisé en ce que la réaction est effectuée dans un milieu de réaction consistant en eau et au moins un solvant organique miscible à l'eau de la classe des éthers cycliques, des éthers de glycols, des amides, des alcools aliphatiques et/ou des sulfoxydes et en présence de morpholine et/ou d'un amino-alcool, et est suivie d'une mesure de la variation d'extinction.

3. Procédé selon la revendication 2, caractérisé en ce que l'on utilise l'eau et le solvant organique dans un rapport de 1 : 4 à 1 : 6.

**Revendications** (pour l'Etat contractant AT)

1. Procédé pour l'analyse des ions potassium dans des liquides à l'aide de composés de formule générale I

(voir dessin p. 15)

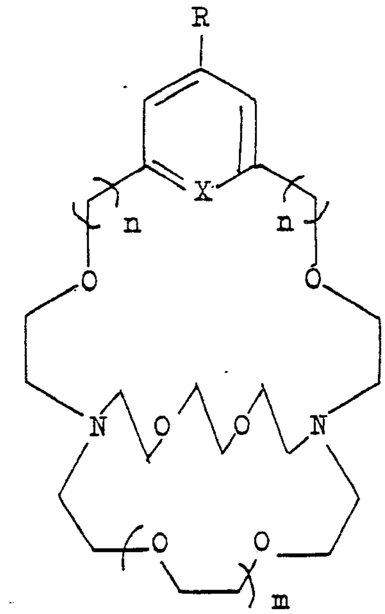

(I)

dans laquelle
n et m = 0 ou 1,
X = N ou COH
R =

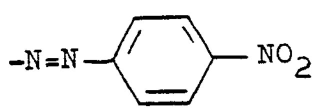

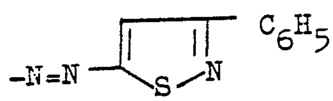

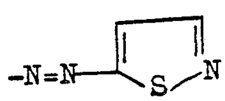

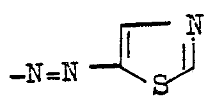

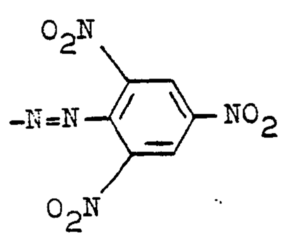

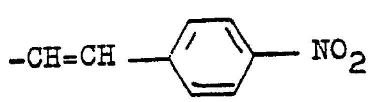

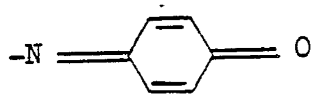

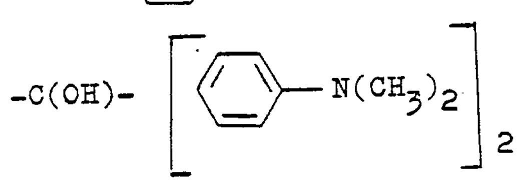

caractérisé en ce que la réaction est effectuée dans un milieu de réaction consistant en eau et au moins un solvant organique miscible à l'eau de la classe des éthers cycliques, des éthers de glycol, des amides, des alcools aliphatiques et/ou des sulfoxydes et en présence de morpholine et/ou d'un aminoalcool et est suivie d'une mesure de la variation d'extinction.

2. Procédé selon la revendication 1, caractérisé en ce que l'on utilise l'eau et le solvant organique dans un rapport de 1 : 4 à 1 : 6.